# EUROPÄISCHE PATENTSCHRIFT

(11) **EP 1 017 665 B1**
(45) Veröffentlichungstag und Bekanntmachung des Hinweises auf die Patenterteilung: **08.01.2003**
(21) Anmeldenummer: 98955346.6
(22) Anmeldetag: 25.09.1998
(51) Int. Cl.: C07C 69/72, C09D 11/02

(54) **ZUSAMMENSETZUNGEN ENTHALTEND ALUMINIUMALKYLACETOACETAT-VERBINDUNGEN, DEREN HERSTELLUNG UND VERWENDUNG ALS DRUCKFARBENADDITIVE**
COMPOSITIONS COMPRISING ALUMINIUM ACETOACETATE COMPOUNDS, THE PRODUCTION AND USE THEREOF AS PRINTING INK ADDITIVES
COMPOSITIONS DE COMPOSES D'ACETO-ACETATE D'ALUMINIUM-ALKYLE, LEUR PRODUCTION ET LEUR UTILISATION COMME ADDITIFS POUR ENCRES D'IMPRIMERIE

(30) Priorität: 27.09.1997 DE 19742828
(43) Veröffentlichungstag der Anmeldung: 12.07.2000
(73) Patentinhaber: SASOL Germany GmbH, 22297 Hamburg (DE)
(72) Erfinder: FINMANS, Peter, D-47199 Duisburg (DE); DIBLITZ, Christina, D-22869 Schenefeld (DE)
(74) Vertreter: Schupfner, Georg U.
(86) Internationale Anmeldenummer: DE9802860
(87) Internationale Veröffentlichungsnummer: WO99016739

(56) Entgegenhaltungen:
- DD-A- 269 142
- GB-A- 772 144
- US-A- 4 264 370
- US-A- 4 329 269

## Beschreibung

Gegenstand der Erfindung sind Zusammensetzungen enthaltend Aluminiumalkylacetoacetat - Verbindungen, deren Herstellung und Verwendung als Druckfarbenadditive.

Die Verwendung von Aluminiumtrisacetylacetonat und Aluminiumtrisethylacetoacetat in Acrylatklebern, Siliconharzen, Tiefdruckfarben u. ä. als Komponente ist bekannt. Die US 4,221,593 offenbart z.B. die Verwendung von Aluminiumdiisopropoxidmonoethylacetoacetat und Aluminiumtrisethylacetoacetat als Geliermittel für Farbbindemittel. Durch Aluminiumtrisacetylacetonat und Aluminiumtrisethylacetoacetat kann bei entsprechender Reaktionsführung eine Vernetzung des jeweiligen Materials erzielt werden. Solchermaßen behandelte Systeme zeigen anwendungstechnische Vorteile. Sie weisen eine verbesserte Rheologie, höherer Beständigkeit gegen Umwelt- und Temperatureinflüsse oder eine erhöhte Festigkeit (Härte, Klebeverhalten) auf. Aluminiumtrisacetylacetonat weist Nachteile hinsichtlich der Toxizität, hoher Rohstoffkosten, aufwendiger Synthese und des festen Aggregatzustands und der geringen Löslichkeit in allen üblichen Lösemitteln auf. Auch Aluminiumtrisethylacetoacetat zeigt eine geringe Löslichkeit in allen üblichen Lösemitteln und eine starke Tendenz zur Ausfällung fester Bestandteile durch Kristallisationsphänomene.

Die US 4,264,370 und die GB-A-772 144 offenbaren Gemische enthaltend Glykol-Verbindungen bzw. Polyalkylenglykole und Aluminium-Verbindungen, wobei die Aluminium-Verbindungen durch Umsetzung von Aluminiumalkoholaten mit unterstöchiometrischen Mengen an β - Keto-Carboxylaten hergestellt sind. Gegenstand der US 4,264,370 und die GB-A-772 144 sind Aluminium-Verbindungen, die stets zumindest eine Alkoholat-Gruppe enthalten.

Aufgabe der Erfindung ist es, Aluminium-Verbindungen zu entwickeln, die alle oder zumindest die meisten der vorbeschriebenen Nachteile nicht aufweisen. Insbesondere sollen die Verbindungen als Additive für Druckfarben geeignet sein und demzufolge eine hohe Verträglichkeit mit Druckfarbenbindemitteln insbesondere für den Offsetdruck aufweisen.

Überraschend wurde festgestellt, daß sich hierfür Aluminium-Verbindungen in bestimmten Lösungsmitteln eignen. In den erfindungsgemäßen Zusammensetzungen sind solche Aluminium-Verbindungen enthalten, die 3 Liganden der folgenden Art aufweisen: 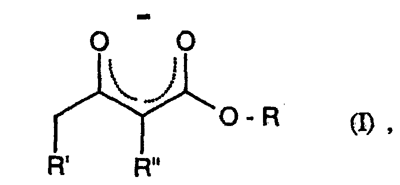 worin
- R für: einen C1- bis C12-, vorzugsweise C1- bis C4-, Kohlenwasserstoff-Rest steht, der weiterhin 1 bis 4, vorzugsweise 1 bis 2, Ether-Bindungen und/oder eine. Hydroxygruppe aufweisen kann. Insbesondere bevorzugt ist für R ein C1- bis C2- AlkylRest und
- R' und R": stehen, unabhängig voneinander, für H und/oder einen C1- bis C4- Alkylrest, vorzugsweise für H und/oder einen C1- bis C2- Alkylrest.

Weiterhin sind Bestandteil der Zusammensetzung nachstehend definierte Glykolether-Verbindung (B) zu mindestens 5 Gew.%, bezogen auf die Summe der Komponenten (A) und (B).

Besonders geeignete Aluminium-Verbindungen sind Aluminium-tris(methylacetoacetat) II ( = Aluminium-Komplex des 3-Oxo-butansäuremethylesters [97494 -08-1]), Aluminium-tris(ethylacetoacetat) III [15306-17-9]

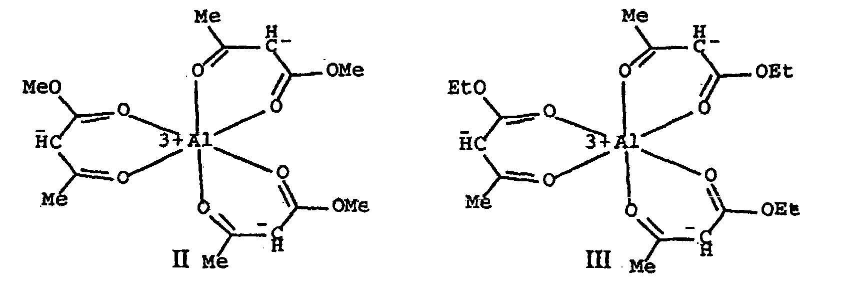

Aluminium-tris(2-hydroxyethoxyethylacetoacetat), Aluminium-tris(dodecylacetoacetat) und Aluminium-tris(benzylacetoacetat).

Diese AluminiumVerbindungen sind z.B. auf dem Wege der Derivatisierung von Aluminiumalkoholaten, vorzugsweise Aluminiumtriisopropoxid durch eine Ligandenaustausch-Reaktion zugänglich. Hierzu wird Aluminiumtriisopropoxid mit z.B. einer Acetessigsäureester-Verbindung umgesetzt.

Problematisch für den Einsatz dieser Aluminium-Verbindungen insbesondere in der Druckfarbenindustrie ist die Wahl eines geeigneten Lösungsmittels. Verwendbar sind hier Mineralöle, die jedoch den Nachteil aufweisen, daß die Löslichkeit der Aluminium-Verbindungen begrenzt ist und daher nur niedrige Al-Gehalte eingestellt werden können. Geeignete Konzentrationen liegen unter 4 Gew%. Die in der Druckfarbenindustrie häufig genutzten Mineralöle im Siedebereich von 240 bis 310 °C führen in vielen Fällen nicht zu flüssigen Produkten.

Weiterhin muß bei der Auswahl des Lösungsmittels darauf geachtet werden, daß in einigen Verarbeitungsprozessen der Druckfarbenindustrie Verarbeitungstemperaturen bis in den Bereich von 200 °C erreicht werden. Der Zusatz niedrig siedender Lösungsmittel wie niederer Alkohole oder ein Überschuß an Acetessigsäureethylester (Ethylacetoacetat), der für diesen Fall in dem Additiv als weitere Rohstoffkomponente enthalten ist, ist damit ausgeschlossen.

Überraschend wurde gefunden, daß bestimmte Glykolether-Verbindungen als Lösemittel für die genannten Aluminium-Verbindungen besonders geeignet sind. Glykolether-Verbindungen im Sinne der Erfindung sind Verbindungen die folgende Struktur aufweisen:

R'''O-(-X-O-)ₙ-H,

worin X , ggf. für jedes n unterschiedlich, für einen gesättigten C2- bis C4-, substituierten oder unsubstituierten Kohlenwasserstoff steht, der an (einem) beliebigen Kohlenstoff-Atome(n) verknüpft sein kann und fakultativ z.B. eine weitere oder mehrere weitere -O- Bindungen (z.B. als =O, -OH oder -OR''' -Gruppe) tragen kann. n steht für eine ganze Zahl von 2 bis 8, besonders bevorzugt 2 bis 4.

Die Glykolether-Verbindungen weisen vorzugsweise ausschließlich Kohlenstoff-, Wasserstoff- und Sauerstoff-Atome auf, können ggf. aber auch ein weiteres anderes Atom pro Molekül aufweisen. Die Endgruppen der Glykolether-Verbindungen sind -H und -OR''' Gruppen. Die Seitenketten können -H, -OR''', -R''' und -OH Gruppen sein.

R''' steht für eine C1- bis C18-, bzw. C1- bis C6-, besonders bevorzugt C2- bis C4-, Kohlenwasserstoff-Gruppe, vorzugsweise eine Alkyl-Gruppe.

Weiterhin kann die Glykolether-Verbindung ggf. auch eine C=O Bindung (pro Molekül), z.B. in Form einer Ester-Bindung (z.B. als -COOR''' oder -OOCR''') aufweisen. Die Glykolether-Verbindungen können z. B. auch alkylierte oder ethoxylierte Zuckermoleküle sein. Vorzugsweise weist die Glykolether-Verbindung ein Molekulargewicht von 60 bis 600 g/mol, besonders bevorzugt von 120 bis 400 g/mol auf.

Besonders gut eignen sich Diethylenglykol-mono-n-butylether (DEnB) und insbesondere der höher siedende Dipropylenglykol-mono-n-butylether (DPnB).

Die Aluminium-Verbindung (A) ist vorteilhafterweise zu mindestens 50 Gew.%, besonders bevorzugt zu mindestens 75 Gew.% in der erfindungsgemäßen Zusammensetzung, bezogen auf die Summe der Komponenten (A) und (B), enthalten. Weiterhin wird die Aluminium-Verbindung (A) vorzugsweise in einer solchen Konzentration eingesetzt, daß ein Aluminiumgehalt der Zusammensetzung von mindestens 3 Gew.% resultiert. Die Glykolether-Verbindung (B) ist zu mindestens 5 Gew.%, besonders bevorzugt zu mindestens 10 Gew.%, jeweils bezogen auf die Summe der Komponenten (A) und (B), in der Zusammensetzung enthalten.

Als besonders vorteilhaft hat es sich erwiesen, wenn das Lösungsmittel zu Beginn der Synthese, also z.B. vor der Zugabe des Alkylacetoacetat-Derivates, anwesend ist bzw kurz nach der Zugabe des Alkylacetoacetat-Derivates zugegeben wird. Als weitere Maßnahme hat es sich als vorteilhaft erwiesen, die Reaktion bei Temperaturen von über 140°C, besser über 160°C und insbesondere über 190°C, zu führen. Über diese Parameter hinaus ist vorzugsweise eine Mindestsynthesezeit von 5 h (bei z.B. über 190 °C) einzuhalten. In dieser Zeit finden eine Reihe von Umesterungs- und Zersetzungsreaktionen statt, die durch die Komplexität des entstehenden Gemisches offenbar eine Kristallisation des Produkts verhindern. Die Reinigung der Produkte kann durch Filtration, ggf. nach Zusatz von Filtrationsmitteln auf Basis von silikatischen Produkten oder Aktivkohle erfolgen.

So erhaltene Produkte sind lagerstabil in Gegenwart von Glas, Metall oder Kunststoffen oder unter sonstigen z.B. durch Umwelt- oder Temperatureinflüsse bedingten Faktoren.

Insbesondere reagiert die erfindungsgemäßen Zusammensetzungen auf Verunreinigungen wie Schmutz, Wasser, Rost (Korrosion im Innern des unbeschichteten Fasses) nicht unter Bildung von Kristallen, auch dann nicht, wenn das Gebinde geöffnet wurde und dann unverschlossen stehenblieb. Hierbei wird weniger die Hydrolysereaktion mit der Luftfeuchtigkeit (eine Reaktion mit den Oberschichten des Produkts mit Wasser führt nur zu einer relativ geringen Beschleunigung des Kristallisationsprozesses) als vielmehr die in der Luft enthaltenen Staubpartikel als Auslöser für den Kristallisationsprozeß verantwortlich gemacht.

Die auf diese Weise hergestellten Produkte weisen die für Al-Alkoholat-Derivate einzigartige und überraschende Eigenschaft auf, über einen Zeitraum von mehr als 6 Monaten an der Luft lagerbar zu sein, ohne daß eine Trübung des Produkts durch Ausbildung von Hydrolyseprodukten oder Produktkristallen festzustellen ist. Das Produkt ist auch nach dieser Lagerung fließfahig und weist einen Al-Gehalt auf, der nur unwesentliche Abweichungen gegenüber dem Zustand zu Beginn der Lagerung aufweist.

Zusammengefaßt weist das erfindungsgemäße Produkt folgende Eigenschaften auf:
- Einzigartige Stabilität gegenüber Hydrolyse
- Flüssiger Aggregatzustand trotz ungewöhnlich niedriger Lösemittelkonzentration
- Außergewöhnliche Eigenschaften sowohl in Bezug auf die Nutzbarkeit für UV/EB-härtende Druckfarbenbindemittel, als auch für Offset-Bindemittel auf Basis von Alkydharzen, Kohlenwasserstoffharzen und/oder modifizieten Kolophonium-Harzen.

Die Rheologie von Druckfarben-Bindemitteln wird in der Regel durch die erfindungsgemäße Zusammensetzung über eine Wechselwirkunung z.B. in der Form einer Verknüpfung/Vernetzung von COOH- oder OH-funktionellen Gruppen mit der Aluminium-Verbindung eingestellt. Ist das Angebot an derartigen funktionellen Gruppen groß, reichen wenige Al-Zentren (0,5 bis 2, vorzugsweise etwa 1, Al-Atom je Summe aus COOH-Gruppen und -OH Gruppen bei Säurezahlen von bis ca. 10 mg KOH/g) aus. Ist die Säurezahl (und die OH-Zahl) des zu verdickenden Harzes deutlich niedriger als 10 mg KOH/g, muß zum Erzielen des gleichen Effekts eine höhere Konzentration des aluminiumhaltigen Derivats vorliegen (1 bis 15 Al-Atome je Summe aus -COOH- + -OH - Gruppen, um das Gleichgewicht d.h. die Wahrscheinlichkeit einer Wechselwirkung zwischen Al-O- und COOH- bzw. OH-Gruppen zu erhöhen.

Entsprechend wird beobachtet, daß die Konzentration der erfindungsgemäß eingesetzten Aluminium - Verbindungen in COOH- und OH-armen Druckfarbenbindemitteln, wie sie häufig in UV/EB-härtenden Systemen auf Basis von Acrylsäureestern genutzt werden, vorteilhafterweise um den Faktor 5-15 höher liegen sollte, als dies in klassischen Bindemitteln unter Beteiligung von Alkyd- und Kolophoniummodifizierten Phenolharzen üblich ist.

In konventionellen Bindemitteln wie Alkydharzen, Kohlenwasserstoffharzen und Kolophonium-modifizierten Phenolharzen werden Konzentrationen von vorzugsweise 0,3 bis 2 Gew.% der Zusammensetzung als Additiv, besonders bevorzugt 0,5 bis 1,5 Gew% im Bindemittel (entspricht etwa 0,03 bis 0,1 Gew.% Aluminium im Bindemittel), verwendet. In UV/EB-härtenden Druckfarbenbindemitteln werden dagegen höhere Additivkonzentrationen von vorzugsweise 1 bis 10 Gew.% Additiv im Bindemittel (entspricht etwa einem Aluminiumgehalt von 0,06 bis 0,6 Gew.% A1) genutzt, um vernetzte Strukturen aufzubauen.

Da UV/EB-härtende Druckfarbenbindemittel aufgrund ihrer reaktiven Doppelbindungen nur eine begrenzte Temperaturbelastbarkeit aufweisen (bei Überschreiten der thermischen Grenzen droht die vorzeitige Aushärtung durch Polymerisation) ist der Prozeß der rheologischen Modifizierung bei niedrigeren Temperaturen als in klassischen Bindemitteln auf Basis von Alkydharzen und Kolophoniummodifizierten Phenolharzen durchzuführen. Dies führt dazu, daß wenig reaktive, relativ hydrolysestabile Al-Alkoholat-Derivate nicht zu einer hinreichenden Reaktionsgeschwindigkeit führen.

In diesem Zusammenhang ist es überraschend, daß die Anwendung der erfindungsgemäßen Aluminiumalkylacetoacetat-Zusammensetzungen in einem Polyester- oder Acrylsäureester-basierten Bindemittel mit Säure- und OH-Zahlen < 2 mgKOH/g zu den effektivsten Ergebnissen in Bezug auf die notwendige Al-Konzentration und die rheologischen Eigenschaften des Bindemittels führt.

Obwohl die erfindungsgemäße Aluminiumalkylacetoacetat-Zusammensetzungen eine niedrige Reaktivität besitzt, wird in Relation zum Al-Gehalt bei diesem Additiv im untersuchten System eine weit höhere Effektivität erzielt als bei allen anderen Al-Alkoholaten oder deren Derivaten des Standes der Technik.

Die erfindungsgemäß additivierten Druckfarben enthalten weiterhin farbgebende Zusätze wie Ruß, anorganische Pigmente, organische Pigmente und/oder lösliche organische Farbstoffe. Erfindungsgemäß finden die additivierten Druckfarben hauptsächlich im Offsettdruck Verwendung. Solche Druckfarben enthalten weiterhin Polyester- oder Polyacrylsäureester-Verbindungen als Bindemittel, die vorzugsweise zur Vernetzung durch Hitze, elektromagnetische Strahlung, insbesondere UV-Strahlung, oder Elektronenstahl geeignete Gruppen, wie reaktive Doppelbindungen, enthalten. Daneben können Initiatoren für die entsprechende Vernetzungsreaktion in den Druckfarben enthalten sein. Solchermaßen ausgestattete Druckfarben gewährleisten eine sekundenschnelle Trocknung.

Die erfindungsgemäßen Zusammensetzungen sind weiterhin geeignet zur rheologischen Modifizierung von physikalisch trockenen Druckfarben auf Basis von Alkydharzen, modifizierzen Kolophoniumharzen oder Kohlenwasserstoffharzen.

### Beispiele

### Beispiel 1

119,2 g Aluminiumtriisopropoxid (AIP) wurde mit 42,7 g Diethylenglycol-mono-nbutylether (DEnB) vermischt und auf 130 bis 140 °C erhitzt. 227,8 Ethylacetoacetat (EAA) wurde bei Sumpftemperaturen bis 180 °C so zudosiert, daß parallel eine destillative Entfernung von 2-Propanol (IPA) aus dem Reaktionsgemisch erfolgte Die Filtration ergab ein gelborangenfarbenes klares Produkt, das nach Zusatz eines Impfkristalls bei Lagerung an der Luft nach ca. 4 Tagen Kristalle bildete.

### Beispiel 2

419 g AIP wurden bei 100 °C vorgelegt und mit Dipropylenglycol-mono-nbutylether (DPnB) versetzt. Nach Aufheizen auf 130 °C wurde EAA innerhalb von 90 Min. zugesetzt. Gleichzeitig wurde IPA destillativ entfernt. Nach Beendigung der Zugabe wurde destilliert, bis die Kopftemperatur sank. Die Sumpftemperatur erreichte dabei maximal 170 °C. Die anschließende Filtration ergab ein klares gelbes Produkt, das in Gegenwart eines Impfkristalls bei Lagerung an der Luft innerhalb von 4 Wochen Kristalle ausbildete.

### Beispiel 3

1 mol Aluminium-sec.butoxid (ASB) wurde auf ca. 140 °C erhitzt und mit einem Gemisch aus 3 mol Methylacetoacetat (MAA) und 80 g DEnB versetzt. IPA wurde über Kopf abgezogen. Anschließend wird die Sumpftemperatur auf 180 °C erhöht und weiter destilliert. Nach Erreichen der Zieltemperatur wurde abgekühlt und durch Filtration ein gelborangenfarbenes klares Produkt erhalten. In Gegenwart von Impfkristallen und Lagerung an der Luft wurde die Bildung eines Feststoffs nach ca. 4 h beobachtet.

### Beispiel 4

3 mol AIP wurden mit 220 g DPnB versetzt und auf 190 °C erhitzt. 9 mol EAA wurden bei dieser Temperatur über einen Zeitraum von 5 h zugesetzt und zeitgleich IPA durch Destillation aus dem Reaktionsgemisch entfernt. Nach Abkühlen und Filtration wurde ein orangefarbenes klares Produkt mit einer Al-Konzentration von 5,7 Gew.% erhalten, das in Gegenwart von Impfkristallen über einen Zeitraum von 6 Monaten an der Luft gelagert werden konnte, ohne daß eine Abscheidung von Feststoffen beobachtet wurde.

### Beispiel 5

947 g AIP wurden mit 1810 g EAA innerhalb von 1,5 h so zur Reaktion gebracht, daß entstehendes Destillat abgezogen wurde. Nach erfolgter Zugabe wurde die Sumpftemperatur auf 180 °C angehoben, zur Entfernung von Leichtsiedern Vakuum angelegt und auf Filtrationstemperatur abgekühlt. Die Filtration ergab ein Produkt, das in IPA gelöst werden kann. Die hierbei erreichbare Al-Konzentration in lagerstabiler Lösung betrug max. 1 Gew.%.

### Beispiel 6

Das Beispiel 6 wurde entsprechend Beispiel 5 durchgeführt. Im Unterschied aber mit abschließender Lösung in einem Druckfarben-typischen Mineralölschnitt der Siedelage von 260 bis 290 °C statt in IPA. Al-Konzentration über 3 Gew.% führten zu Produkten, die bei Raumtemperatur innerhalb von 24 h Kristalle ausbildeten.

### Beispiel 7

Das Beispiel 7 wurde entsprechend Beispiel 1 durchgeführt. Im Unterschied aber mit der DPnB-Zugabe nach der Reaktion von AIP mit EAA. Das erhaltene Produkt kristallisierte innerhalb weniger Stunden nach Filtration und Abkühlung aus

### Beispiel 8

Ein Standard-Heatset-Firnis auf Basis eines Phenol-modifizierten Kolophoniumharzes (3 Teile) und eines Isophthalsäure-basierten Alkydharzes (2 Teile) sowie eines Mineralöls mit einem Siedebereich von 260 bis 290 °C (3 Teile) wurden bei einer Temperatur von 180 °C mit 0,6 bis 2,5 Gew.% des Produkts aus Beispiel 4 unter intensiver Rührung versetzt und für 15 - 60 Minuten bei dieser Temperatur belassen und anschließend abgekühlt. Das Harzgemisch wies eine Säurezahl von ca. 10 und eine OH-Zahl von ca. 30 auf. Durch die Additivierung entstand aus dem Firnis mit Newton'schem Fließverhalten ein homogenens und strukturviskoses Bindemittel mit Fließgrenze. Die exakten rheologischen Eigenschaften konnten durch geringfügige Variation der Additivkonzentration in der gewünschten Weise eingestellt werden

### Beispiel 9

Ein UV/EB-härtbares Acrylatharz (Säurezahl < 1 mgKOH/g, OH-Zahl < 2 mgKOH/g, Newton'sche Rheologie) wurde in Gegenwart von 1 - 5 Gew.% des in Beispiel 4 erhaltenen Produkts auf eine Temperatur von 100 °C erhitzt. Erhalten wurde ein von starker Strukturviskosität geprägtes Bindemittel für Druckfarben, dessen Rheologie durch entsprechende Einstellung des Al-Gehaltes an die Erfordernisse angepaßt werden konnte.

## Patentansprüche

1. Zusammensetzung enthaltend
(A) eine oder mehrere Aluminium-Verbindungen, die drei Liganden pro Aluminium - Atom der folgenden Art aufweisen: 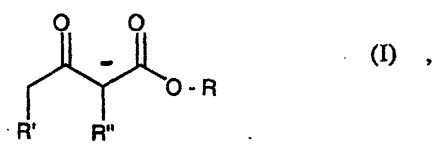 worin
R für einen C1- bis C12- Kohlenwasserstoffrest steht, der weiterhin 1 bis 4 Ether-Bindungen und/oder eine Hydroxygruppe aufweisen kann und
R' und R" ' unabhängig voneinander für H und/oder einen C1- bis C4- Kohlenwasserstoffrest stehen
und
(B) eine oder mehrere Glykolether-Verbindungen, wobei die Glykolether-Verbindungen folgende Struktur aufweisen:
R'''O-(X-O)ₙ-H
worin,
R''' für eine C1- bis C18-Kohlenwasserstoff-Gruppe steht und
n eine ganze Zahl von 2 bis 8 steht und
X ggf. für jedes n unterschiedlich, für einen gesättigten, substituierten oder unsubstituierten C2- bis C4- Kohlenwasserstoff steht, der an beliebigen Kohlenstoff-Atomen verknüpft sein kann und fakultativ eine weitere oder mehrere weitere -O- Bindungen, z.B. als =O, -OH oder -OR''' Gruppe, tragen kann,
und die Glykolether-Verbindung (B) zu mindestens 5 Gew.%, bezogen auf die Summe der Komponenten (A) und (B), in der Zusammensetzung enthalten ist.

2. Zusammensetzungen gemäß Anspruch 1, **dadurch gekennzeichnet, daß** die Aluminium-Verbindung (A) zu mindestens 50 Gew%, vorzugsweise zu mindestens 75 Gew.%, jeweils bezogen auf die Summe der Komponenten (A) und (B), in der Zusammensetzung enthalten ist.

3. Zusammensetzungen gemäß einem der vorhergehenden Ansprüche, **dadurch gekennzeichnet, daß** die Aluminium-Verbindung Aluminium-tris(methylacetoacetat) und/oder Aluminium-tris(ethylacetoacetat) ist.

4. Zusammensetzung gemäß einem der vorhergehenden Ansprüche, **dadurch gekennzeichnet, daß** die Glykolether-Verbindung Dipropylenglykol-mono-n-butylether und/oder Diethylenglykol-mono-n-butylether ist.

5. Zusammensetzung gemäß einem der vorhergehenden Ansprüche, **dadurch gekennzeichnet, daß** die Zusammensetzung zusätzlich Polyester- oder Polyacrylsäureester-Verbindungen enthält.

6. Zusammensetzung gemäß einem der vorhergehenden Ansprüche, **dadurch gekennzeichnet, daß** die Zusammensetzung zusätzlich farbgebende Zusätze wie Ruß, anorganische Pigmente, organische Pigmente und/oder löslich organische Farbstoffe enthält.

7. Verfahren zur Herstellung der Zusammensetzung gemäß einem der vorhergehenden Ansprüche, **dadurch gekennzeichnet, daß** man die Zusammensetzung durch Umsetzung eines C1- bis C12-Aluminiumalkoholates mit einer 3-Oxo-Carbonsäuereester-Verbindung bei Temperaturen von über 140°C, vorzugsweise über 160°C in Gegenwart einer Glykolether-Verbindung herstellt.

8. Verfahren gemäß Anspruch 7, **dadurch gekennzeichnet, daß** man das Produkt / die Zusammensetzung über 1 bis 10 h, vorzugsweise über 4 bis 8 h, während oder nach der Umsetzung bei über 140°C hält.

9. Zusammensetzung herstellbar nach einem der Verfahren gemäß den Ansprüchen 7 bis 8.

10. Verwendung der Zusammensetzung gemäß den Ansprüchen 1 bis 6 und 9 als Additiv für farbgebende Zusammensetzungen.

11. Verwendung gemäß Anspruch 10 als Druckfarbenadditiv.

12. Verwendung gemäß Anspruch 10 als Additiv für strahlungs- oder elektronenstrahl-härtende Druckfarben.

13. Verwendung gemäß einem der Ansprüche 10 bis 12, **dadurch gekennzeichnet, daß** die Zusammensetzung in einer Konzentration von 0,2 bis 10 Gew.%, bezogen auf das Bindemittel in der farbgebenden Zusammensetzung eingesetzt wird.

## Claims

1. A composition containing
(A) one or more aluminium compounds comprising three ligands of the following type per aluminium atom: 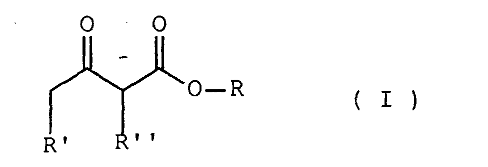 where
**R** represents a C₁- to C₁₂-hydrocarbon residue which can furthermore comprise 1 to 4 ether bond(s) and/or one hydroxy group and
**R'** and **R"** are independently of one another H and/or a C₁- to C₄-hydrocarbon residue
and
(B) one or more glycol ether compound(s) having the following structure:
**R'''O-(X-O)**_{**n**}**-H**
where
**R'''** is a C₁- to C₁₈-hydrocarbon group and
**n** represents an integer from 2 to 8 and
**x** which can be different for each **n** represents a saturated, substituted or unsubstituted C₂- to C₄-hydrocarbon that can be linked to any carbon atoms and can optionally have one or several additional -O- bond(s), for example as =O, -OH, or -OR''' group
and said composition contains at least 5 percent by weight of the glycol ether compound (B), based on the total of components (A) and (B).

2. Compositions according to claim 1,
**characterised in that** the composition contains at least 50 percent by weight, preferably at least 75 percent by weight of the aluminium compound (A), each referring to the total of components (A) and (B).

3. Compositions according to any one of the preceding claims,
**characterised in that** the aluminium compound is aluminium-tris(methylacetoacetate) and/or aluminium-tris(ethylacetoacetate).

4. A composition according to any one of the preceding claims,
**characterised in that** the glycol ether compound is dipropylene glycol-mono-n-butyl ether and/or diethylene glycol-mono-n-butyl ether.

5. A composition according to any one of the preceding. claims,
**characterised in that** the composition additionally contains polyester- or polyacrylic ester compounds.

6. A composition according to any one of the preceding claims,
**characterised in that** the composition additionally contains colouring additives, such as soot, inorganic pigments, organic pigments, and/or soluble organic dyes.

7. A process for producing a composition according to any one of the preceding claims,
**characterised in that** the composition is produced by reacting a C₁- to C₁₂-aluminium alcoholate with a 3-oxo-carboxylic ester compound at temperatures of higher than 140 °C, preferably higher than 160 °C, in the presence of a glycol ether compound.

8. The process of claim 7,
**characterised in that** the product/composition is maintained at >140 °C for 1 to 10 hour(s), preferably 4 to 8 hours, during or after the reaction.

9. A composition which can be produced by a process according to claims 7 to 8.

10. Use of the composition according to claims 1 to 6 and 9 as an additive for colouring compositions.

11. The use according to claim 10 as a printing ink additive.

12. The use according to claim 10 as an additive for radiation-curing or electron-beam-curing printing inks.

13. The use according to any one of claims 10 to 12,
**characterised in that** the composition is employed in concentrations of from 0.2 to 10 percent by weight, based on the bonding agent quantity in the colouring composition.

## Revendications

1. Composition comprenant
(A) une ou plusieurs liaisons d'aluminium, qui présentent trois ligands par atome d'aluminium du type suivant : 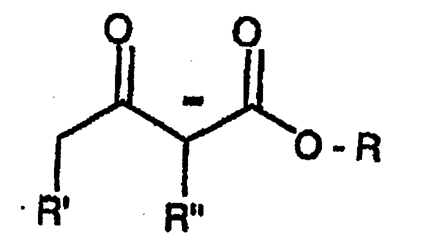 où
R représente un reste d'hydrocarbure C1- à C12-, qui peut présenter de plus 1 à 4 liaisons d'éther et/ou un groupe hydroxy et
R' et R'' représentent indépendamment l'un de l'autre H et/ou un reste d'hydrocarbure C1- à C4-
et
(B) une ou plusieurs liaisons de glycoléther, les liaisons de glycoléther
présentant la structure suivante :
R'''O-(X-O)ₙ-H
où
R''' représente un groupe hydrocarbure C1- à C18- et
n représente un nombre entier de 2 à 8 et
X le cas échéant différent pour chaque n, représente un hydrocarbure saturé, substitué ou non substitué C2- à C4-, qui peut être lié à n'importe quels atomes de carbone et peut facultativement supporter une ou plusieurs autres liaisons -O, par exemple comme groupe =O, -OH ou -OR'',
et où la liaison de glycoléther (B) est contenue pour au moins 5 % du poids, en référence à la somme des composants (A) et (B), dans la composition.

2. Composition selon la revendication 1, **caractérisée en ce que** la liaison d'aluminium (A) est contenue pour au moins 50 % en poids, de préférence pour au moins 75 % en poids, respectivement en référence à la somme des composants (A) et (B), dans la composition.

3. Composition selon une des revendications précédentes, **caractérisée en ce que** la liaison d'aluminium est du tris(méthylacétoacétate) d'aluminium et/ou du tris(éthylacétoacétate) d'aluminium.

4. Composition selon une des revendications précédentes, **caractérisée en ce que** la liaison de glycoléther est du dipropylèneglycol-mono-n-butyléther et/ou du diéthylèneglycol-mono-n-butyléther.

5. Composition selon une des revendications précédentes, **caractérisée en ce que** la composition contient en plus des liaisons d'ester d'acide de polyester ou de polyacrylique.

6. Composition selon une des revendications précédentes, **caractérisée en ce que** la composition contient en plus des additifs colorants comme de la suie, des pigments anorganiques, des pigments organiques et/ou des colorants organiques solubles.

7. Procédé pour la fabrication de la composition selon une des revendications précédentes, **caractérisée en ce que** l'on fabrique la composition par transposition d'un alcoolat d'aluminium C1- à C12- avec une liaison d'ester d'acide carboxylique 3-oxo à des températures supérieures à 140 °C, de préférence supérieures à 160 °C en présence d'une liaison de glycoléther.

8. Procédé selon la revendication 7, **caractérisé en ce que** l'on maintient le produit/la composition pendant 1 à 10 h, de préférence pendant 4 à 8 h, à plus de 140 °C pendant ou après la transposition.

9. Composition pouvant être élaborée selon un des procédés des revendications 7 à 8.

10. Utilisation de la composition des revendications 1 à 6 et 9 comme additif pour les compositions colorantes.

11. Utilisation selon la revendication 10 comme additif d'encre d'imprimerie.

12. Utilisation selon la revendication 10 comme additif pour les encres d'imprimerie durcissant au rayonnement ou aux rayons électroniques.

13. Utilisation selon une des revendications 10 à 12, **caractérisée en ce que** la composition est utilisée dans une concentration de 0,2 à 10 % en poids, en référence au liant, dans la composition colorante.
